# EUROPEAN PATENT SPECIFICATION

(11) **EP 1 865 933 B1**
(45) Date of publication and mention of the grant of the patent: **04.11.2015**
(21) Application number: 06749146.4
(22) Date of filing: 30.03.2006
(51) Int. Cl.: A61K 9/70, A61K 31/165, A61P 29/00

(54) **LOW-CONCENTRATION CAPSAICIN PATCH AND METHODS FOR TREATING NEUROPATHIC PAIN**
CAPSAICIN-PFLASTER MIT GERINGER KONZENTRATION UND VERFAHREN ZUR BEHANDLUNG VON NEUROPATHISCHEM SCHMERZ
TIMBRE TRANSDERMIQUE A FAIBLE CONCENTRATION DE CAPSAICINE ET PROCEDES DE TRAITEMENT DES DOULEURS NEUROPATHIQUES

(30) Priority: 30.03.2005 US 666880 P
(43) Date of publication of application: 19.12.2007
(73) Proprietor: Vanderbilt Royalty Sub L.P., Stamford, CT 06901 (US)
(72) Inventor: BLEY, Keith, R., Mountain View, CA 94043 (US)
(74) Representative: Roques, Sarah Elizabeth
(86) International application number: PCT/US2006/012271
(87) International publication number: WO 2006/105481

(56) References cited:
- EP-A- 1 568 365
- WO-A-98/53825
- WO-A-2004/089361
- US-A- 5 665 378
- US-A- 5 869 533
- US-A1- 2001 002 406

## Description

### RELATED APPLICATIONS

### FIELD

The patches and methods described here are in the field of dermal drug delivery, and specifically, dermal delivery of capsaicin or a capsaicin analog for the treatment of neuropathic pain.

### BACKGROUND

Almost four million people in the United States are afflicted with neuropathic pain syndromes (Bennett GJ. Neuropathic pain: new insights, new interventions. Hosp Pract. 1998 Oct. 15; 33(10):95-8), and the prevalence is rising (Dworkin RH. An Overview of Neuropathic Pain: Syndromes, Symptoms, Signs, and Several mechanisms. Clin JPain. 2002 Nov-Dec; 18(6):343-9). Neuropathic pain may last many years and can even be permanent. Due to poor efficacy of existing medicines, neuropathic pain has a devastating impact on patients' quality of life and high societal costs (Harden N, Cohen M. Unmet Needs in the Management of Neuropathic Pain. J Pain Symptom Management. 2003. 25(5 Suppl):S12-S17). Causes of neuropathic pain are highly diverse and include reactivation of latent viruses, direct trauma to nerves, diabetes, and HIV-infection and therapy *(id.).*

Currently approved treatments for post-herpetic neuralgia (PHN) are oral gabapentin (Rowbotham M, Harden N, Stacey B, Bernstein P, Magnus-Miller L. Gabapentin for the Treatment of Postherpetic Neuralgia: a Randomized Controlled Trial. JAMA. 1998 Dec 2; 280(21):1837-42; Rice AS, Maton S; Postherpetic Neuralgia Study Group. Gabapentin in Postherpetic Neuralgia: A Randomised, Double Blind, Placebo Controlled Study. Pain. 2001. 94:215-24) and the topical lidocaine patch (Rowbotham MC, Davies PS, Verkempinck C, Galer BS. Lidocaine Patch: Double-Blind Controlled Study of a New Treatment Method for Post-Herpetic Neuralgia. Pain. 1996. 65:39-44; Galer BS, Jensen MP, Ma T, Davies PS, Rowbotham MC. The Lidocaine Patch 5% Effectively Treats All Neuropathic Pain Qualities: Results of a Randomized, Double-Blind, Vehicle-Controlled, 3-week Efficacy Study with use of the Neuropathic Pain Scale. Clin J Pain. 2002 Sep-Oct; 18(5):297-301). Both have shown efficacy, but they only led to partial pain relief in a subset of patients. Gabapentin appears to be better tolerated than other anticonvulsants, but CNS-related side effects such as somnolence and dizziness, as well as the need for dose-titration and three times daily dosing, frequently limit its use in some patients (Rowbotham MC, Davies PS, Verkempinck C, Galer BS. Lidocaine Patch: Double-Blind Controlled Study of a New Treatment Method for Post-Herpetic Neuralgia. Pain. 1996 Apr; 65(1):39-44). There are no FDA-approved pain medicines specifically for painful HIV-associated neuropathy. Speaking generally in the field of neuropathic pain management, even with the recent approvals of Lyrica^{®} (pregabalin) and Cymbalta^{®} (duloxetine), there remains a significant medical need for a therapeutic modality with substantial efficacy and a favorable side effect profile.

Capsaicin, the pungent ingredient in chili peppers, activates vanilloid receptors (TRPV1) expressed in cutaneous nociceptive sensory nerve fibers, leading acutely to burning pain sensations followed by prolonged functional inactivation of these nociceptors (Caterina MJ, Julius D. The Vanilloid Receptor: a Molecular Gateway to the Pain Pathway. Annu. Rev. Neurosci. 2001. 24:487-517). Topical low-concentration capsaicin creams have shown efficacy in PHN in controlled clinical trials, but their practical use has been hampered by the inconvenience of multiple daily applications needed to produce efficacy. Moreover, each cream application is often associated with painful burning sensations (Hautkappe M, Roizen MF, Toledaon A, Roth S, Jeffries JA, Ostermeier AM. Review of the Effectiveness of Capsaicin for Painful Cutaneous Disorders and Neural Dysfunction. Clin. J. Pain. 1998. 14:97-106).

In uncontrolled compassionate use in various neuropathic pain syndromes, one-time applications of high-concentration capsaicin creams (e.g., 5 - 10% w/w) have shown promising pain relief (Robbins WR, Staats PS, Levine J, et al. Treatment of Intractable Pain with Topical Large-Dose Capsaicin: Preliminary Report. Anesth. Analg. 1998. 86:579-583). Patches with a high concentration (e.g., 8% w/w) of capsaicin have also undergone clinical evaluation *(See:* D Simpson, S Brown, S Chang, J Jermano and C107 Study Group. Controlled Study of High-Concentration Capsaicin Patch for Painful HIV-Associated Distal Sensory Polyneuropathy. 2006. 13th Conference on Retroviruses and Opportunistic Infections). Unfortunately, the high-concentration capsaicin creams described above displayed very high levels of pungency, often requiring patients to undergo regional nerve blocks in order to tolerate the treatment procedure (Robbins *et al.,* 1998). High-concentration capsaicin patches display lower levels of pungency, but still induce a substantial percentage of patients to ask for opioid analgesics during and/or following treatment procedures.

Accordingly, it would be desirable to have low-concentration capsaicin patches for the treatment of neuropathic pain, as they may be better tolerated than high-concentration patches and high-concentration capsaicin creams.

### SUMMARY

Described herein are patches and methods for treating neuropathic pain. In general, the neuropathic pain-relieving patches include capsaicin or a capsaicin according to claim 1 analog at a concentration of less than about 1% by weight and a penetration enhancer wherein the penetration enhancer is diethylene glycol monoethyl ether. The patches are formulated to relieve pain for a sustained time period of at least about one month, for example, at least about two months, or at least about three months or more.

The penetration enhancer suitable for use in the neuropathic pain-relieving patches is diethylene glycol monoethyl ether.

The pain-relieving patches typically include a self-adhesive matrix, but any polymeric matrix may be employed, so long as the patch is capable of delivering capsaicin or a capsaicin analog and relieving neuropathic pain over the desired time period. In one variation, the self-adhesive matrix includes an amine-resistant polysiloxane. In this particular variation, it may be desirable to incorporate a silicone oil to the matrix. In another variation, the self-adhesive matrix includes polyisobutylene adhesives in combination with plasticizer which is mineral oil. In yet another variation, adhesive can be acrylate-based whereby co-polymers of alkyl acrylates with acrylamide or acetonitrile. Such polymers can range from C₄ to C₈.

Also described here are methods for treating neuropathic pain. The general method includes applying a neuropathic pain-relieving patch that has less than about 1% capsaicin or a capsaicin analog for a period of about 30 minutes, a period of about 60 minutes, but not longer than a period of about 120 minutes.

### BRIEF DESCRIPTION OF THE DRAWINGS

FIG. 1 is a flow diagram showing an exemplary process for manufacturing the patches described herein.
FIG. 2 is a graph showing pooled data from all patients in Studies 1,2 and 3 receiving low-concentration capsaicin patch treatments. Studies 1 and 2 enrolled subjects with PHN. Study 3 enrolled subjects with HIV-AN.

### DETAILED DESCRIPTION

The patches and methods described here treat neuropathic pain by dermally delivering an active agent, i.e., capsaicin or a capsaicin analog. As used herein, the term "dermally" or "dermal" refers to topical delivery of drug mainly to the skin layers with no drug or minimal drug reaching the systemic circulation. The patches generally include a self-adhesive matrix and a backing layer, and less than about 1% by weight capsaicin or a capsaicin analog and a penetration enhancer in the self-adhesive matrix. Clinical data has been generated from patches containing 0.04% w/w capsaicin, and is provided below.

The primary advantage of the low-concentration patch is that tolerability is improved due to reduced pungency. That is, patients exposed to the low-concentration patch will be less likely to ask to have the patch removed during a treatment procedure. They will also likely consume lower amounts of opioid pain relievers in order to deal with treatment-associated pain. As with all topical capsaicin-based pain reducing products, the theory is that hyperactive nociceptors in the skin are pathologically active in patients with peripheral neuropathic pain syndromes. Exposure to capsaicin causes these pathologically hyperactive nerve fibers to cease functioning for an extended period of time; this process is often referred to as 'desensitization' (Bley, K.R. Recent developments in transient receptor potential vanilloid receptor 1 agonist-based therapies. Expert Opin Investig Drugs. 2004. 13:1445-56). Although the low-concentration patch may not provide a degree of average pain relief as great as a high-concentration capsaicin patch, for a subset of patients, the low-concentration patch induces persistent and clinically significant pain reductions without significant side effects.

Active agents. Active agents that may be used in the low-concentration patches include capsaicin, dihydrocapsaicin, nordihydrocapsaicin, homocapsaicin, homodihydrocapsaicin, nonivamide, cis-capsaicin, olvanil, arvanil and analogs of capsaicin such as capsaicin esters and derivatives of the amide side chain.

Penetration enhancers. The penetration enhancer for use in the neuropathic pain-relieving patches is diethylene glycol monoethyl ether.

Maxtrix materials. The pain-relieving patch may comprise self-adhesive matrix, for example, an amine-resistant polysiloxane. In one variation, the amine resistant polysiloxane comprises a mixture of medium and high tack polysiloxane. A silicone oil may be added to the polysiloxane adhesive or mixture thereof. Silicone oil enhances adhesive properties and may constitute from 0.5 to 5% by weight of silicone oil. In another variation, the matrix comprises polyisobutylene adhesives in combination with plasticizer which is mineral oil. In yet another variation, the adhesive can be acrylate-based whereby co-polymers of alkyl acrylates with acrylamide or acetonitrile. Such polymers can range from C₄ to C₈.

Other additives. The neuropathic pain-relieving patch may also comprise a silicone oil, a viscosity increasing agent, a penetration enhancer or a combination thereof. The viscosity increasing agent may be, for example, ethylcellulose, hydropropylcellulose, or mixtures thereof. The penetration enhancer may be, as example, fatty acids (linear or branched), fatty acid esters, organic acids, ethers, amides, amines, hydrocarbons, alcohols, phenols, polyols, fatty alcohols, surfactants (anionic, cationic, nonionic or bile salts), ureas, terpenes (hydrocarbons, alcohols, ketones, oils, oxides).

Backing layer. The backing layer typically is made from a polyester film and generally about 10 to about 20 µm thick. The backing layer may also be made from such materials as ethylene vinyl acetate, polyethylene, polyurethane, pigmented polyethylene plus polyester with/without aluminum vapor coating.

In one variation, the pain-relieving patches include 0.04% by weight or less of capsaicin or a capsaicin analog, 10-20% by weight of diethylene glycol monoethyl ether, 0-2% by weight of ethylcellulose, 0-5% by weight of silicone oil, and 58-85% by weight of a self-adhesive polysiloxane. These patches may also comprise a backing layer, for example, the polyester films mentioned above.

In another variation, the pain-relieving patches comprise capsaicin, or a capsaicin analog, wherein the concentration of the capsaicin or capsaicin analog is less than 1%, and a penetration enhancer, whereby delivery of capsaicin from the patch continues for at least an hour, and whereby a single use of the patch provides a therapeutic benefit for at least one month, two months, or three months. The penetration enhancer is diethylene glycol monoethyl ether. The patches may also comprise a self-adhesive matrix (such as an amine-resistant polysiloxane), a silicone oil, a viscosity increasing agent, and/or a backing layer. In some variations, the viscosity increasing agent is ethylcellulose.

Methods for treating neuropathic pain are also described here. In general, the methods comprise the step of dermally delivering a single administration of capsaicin or a capsaicin analog by topically applying a low-concentration neuropathic pain-relieving patch to any area of the skin affected by neuropathic pain. The patch includes capsaicin or a capsaicin analog at a concentration of less than 1%. In some variations, the step of dermal delivery of the capsaicin or capsaicin analog provides a therapeutic benefit, i.e., significant relief of neuropathic pain, for at least one month, at least two months, or at least three months.

The clinical efficacy observed following single treatments of neuropathic pain patients with low-concentration patches is quite surprising given the requirement of repeated applications or chronic exposure for other low-concentration topical capsaicin products. For instance, it is widely accepted that low-concentration creams (0.025 to 0.1% w/w) must be applied multiple times per day for efficacy to occur. Moreover, efficacy slowly develops over a period of weeks. According to the product label for Zostrix^{®}, a widely used over-the-counter capsaicin-containing cream: "Capsaicin must be used regularly every day as-directed if it is to work properly. Even then, it may not relieve your pain right away. The length of time it takes to work depends on the type of pain you have. In persons with arthritis, pain relief usually begins within 1 to 2 weeks. In most persons with neuralgia, relief usually begins within 2 to 4 weeks, although with head and neck neuralgias, relief may take as long as 4 to 6 weeks. Once capsaicin has begun to relieve pain, you must continue to use it regularly 3 or 4 times a day to keep the pain from returning" (source: http://www.drugs.com/cons/Zostrix.html).

Similarly topical capsaicin-containing patches are also used to treat chronic lower back pain. Several clinical studies with a marketed capsaicin patch (37.4 µg capsaicin per cm²; ABC Lokale Schmerz-Therapie Waerme-Pflaster^{®}) have indicated that pain relief occurs gradually and that the patches must be worn every day. Keitel W, Frerick H, Kuhn U, Schmidt U, Kuhlmann M, Bredehorst A. Capsicum pain plaster in chronic non-specific low back pain. Arzneimittelforschung. 2001. 51(11):896-903. Frerick H, Keitel W, Kuhn U, Schmidt S, Bredehorst A, Kuhlmann M. Topical treatment of chronic low back pain with a capsicum plaster. Pain. 2003. 106(1-2):59-64.

### Patches

The characteristics of the patches used in the methods of the present invention are described in Table 1.

**Table 1: Characteristics of the Low-Concentration Capsaicin Patch**

| **Material** | **Grade** | **Function** | **Weight (mg)** |
|---|---|---|---|
| **Drug Matrix** | | | |
| *trans*-Capsaicin | cGMP Manufactured | Active Ingredient | 179.0 |
| Diethylene Glycol Monoethyl Ether (e.g., Transcutol^{®}) | USP26/NF21 DMF 5718 | Solubilizer | 430.0 |
| Silicone Adhesive (amine resistant, BIO-PSA 4301) | DMF 7114 | Adhesive | 470.0 |
| Silicone Adhesive (amine resistant, BIO-PSA 4201) | DMF7114 | Adhesive | 1098.0 |
| Silicone Oil, 12,500 cSt | USP26/NF21 for Dimethicone | Plasticizer | 45.0 |

| **Formulation Aids (Removed During Drying)** | | | |
|---|---|---|---|
| n-Heptane | Merck extra pure | Solvent for Adhesive | NA |
| Ethyl Cellulose N 50 | USP26/NF21 | Thickener | 17.9 |

| **Backing Layer** | | | |
|---|---|---|---|
| Polyethylene Terephthalate (PET) Film (matte, inner side siliconized) | DMF 7373 | Backing Layer | 694.4-887.6 |
| e.g. Hostphan MN 19 | 21 CFR | | |
| Thickness: 19 µm | 177.1630 | | |

| **Removable Protective Layer (Release Liner)** | | | |
|---|---|---|---|
| Polyester film, fluoropolymer-coated. E.g., Scotchpak 1022 | DMF 2610-E | Protective | 1988.0-3220.0 |
| Thickness: 76.2 µm | | | |

Capsaicin is dissolved in a mixture of solubilizer and thickener. The adhesives and silicone oil are then added with a solvent. This mixture is dispersed, and the homogenized adhesive mass is coated onto a removable protective film liner. After removal of the solvent and drying, the matrix film is then laminated onto a backing layer. The laminate is wound into rolls and patches are punched out to the appropriate sizes before being packaged in heat-sealed Barex^{®} pouches.

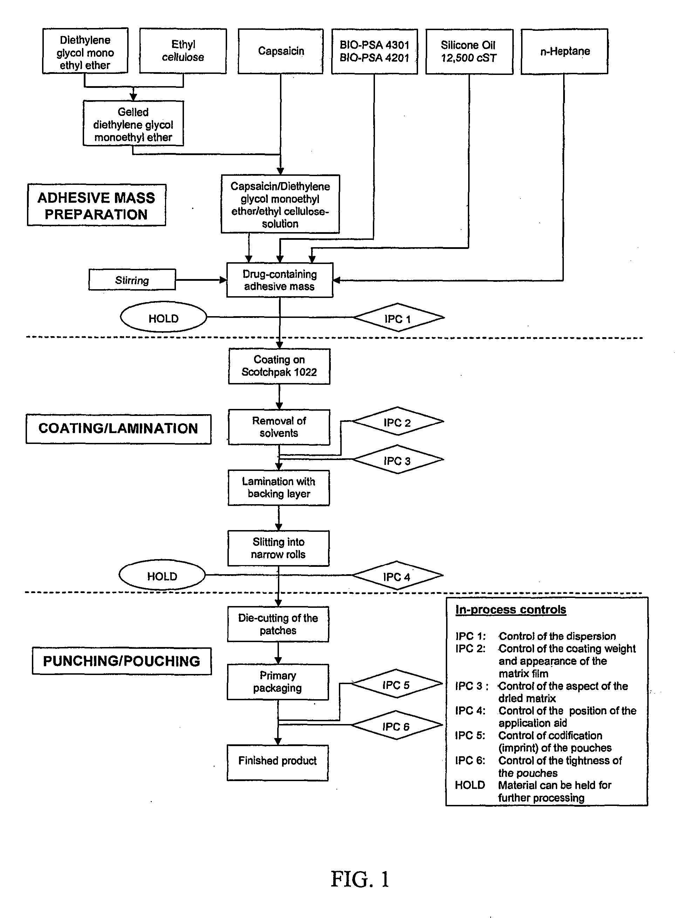

A self-explanatory flow diagram of the manufacturing process is shown in Figure 1.

### Method of Use

The low-concentration capsaicin patches should be applied to the skin of a patient for about one (1) hour. However, this time may be lengthened or shortened depending on the particular patient's needs (e.g., based on the amount and/or severity of pain). Prior to application of the patch, a local anesthetic (e.g., in the form of a topically applied cream or a nerve block) is used to numb the skin of the treatment area. Applying the anesthetic helps ameliorate the sometimes intense burning sensations produced by the application of capsaicin to the skin. The painful area to be treated is defined by a health care provider, and patches are cut to provide complete coverage of the area. Following the approximate one-hour application, patch pieces are removed and residual capsaicin from the treated area is removed with a cleansing gel. Capsaicin patches would be determined to have provided a therapeutic benefit if the pain symptoms reported by the patient prior to treatment were reduced following the treatment procedure.

### EXAMPLES

For all three studies described in the following examples, the diagnoses of either postherpetic neuralgia ("PHN") or painful HIV-associated neuropathy (HIV-AN) was confirmed by medical history and physical examination. Patients who met the inclusion/exclusion criteria then completed a pain intensity diary for at least five days. Those continuing to meet eligibility criteria were randomized and treated.

The treatment procedure in all three studies began with application of a 4% w/w lidocaine-based topical cream to the skin treatment area for one hour. Subsequently, the anesthetic cream was removed and patches which had been cut to fit the treatment area were applied for one hour. After patch removal, a cleansing gel was applied for one minute and then wiped off. In case of significant pain during or immediately following patch application, an oral oxycodone elixir was available. In the PHN clinical studies, subjects were given 30 tablets of hydrocodone/acetaminophen (5 mg/500 mg) to be taken as needed for any procedure-related pain during the first five days after treatment. Patients were monitored for approximately three hours after patch removal and released. They were seen again at follow-up visits.

### EXAMPLE 1 - STUDY 1

Because of its chronic and stable time course, and its occurrence in otherwise healthy and active individuals, postherpetic neuralgia (PHN) is a preferred clinical model of neuropathic pain for the initial study of new therapeutic modalities.

### Study Population

A total of 299 patients with PHN that persisted at least 6 months after crusting of vesicles, without significant pain of other origin, were enrolled. The area of worst pain was less than 1000 cm² and did not include the face. Average pain intensity, rated by the patient twice daily on an 11-point scale (0 = no pain, 10 = worst pain imaginable), was between 3 and 8 during the screening period. Concomitant use of non-topical chronic pain medication was permitted, as long as the regimen remained stable for 3 weeks before treatment and throughout the study.

### Study Design

Patients were randomized in a 3:3:3:1:1: ratio to receive either high-concentration (640 µg/cm² capsaicin) patches for durations of 30, 60 or 90 minutes or low-concentration (3.2 µg/cm² capsaicin) patches for 30, 60 or 90 minutes. The high-concentration and low-concentration patches were of identical appearance. Patients, investigators and sponsor staff were blinded to the treatment received until all data collection activities were completed. Because of prior reports that single applications of low-concentration capsaicin applications do not cause reductions in neuropathic pain, these low-concentration patches were deemed unlikely to exert a significant therapeutic effect.

### EXAMPLE 2 - STUDY 2

### Study Population

A total of 155 patients with PHN that persisted at least 3 months after crusting of vesicles, without significant pain of other origin, were enrolled. The area of worst pain was less than 1000 cm² and did not include the face. Average pain intensity, rated by the patient twice daily on an 11-point scale (0 = no pain, 10 = worst pain imaginable), was between 3 and 8 during the screening period. Concomitant use of non-topical chronic pain medication was permitted, as long as the regimen remained stable for 3 weeks before treatment and throughout the study.

### Study Design

PHN patients were randomized in a 2:1 ratio to receive either high-concentration (640 µg/cm² capsaicin) or low-concentration (3.2 µg/cm² capsaicin) patches for 60 minutes. The high-concentration and low-concentration patches were of identical appearance. Patients, investigators and sponsor staff were blinded to the treatment received until all data collection activities were completed. Because of prior reports that low concentration capsaicin applications did not cause a sustained reduction in neuropathic pain, these low-concentration patches were deemed unlikely to exert a significant therapeutic effect.

### EXAMPLE 3 - STUDY 3

### Study Design

This double-blind, multi-center study randomized 307 subjects with HIV-AN symptoms ≥ 2 months, stratified by neurotoxic antiretroviral HIV treatment status, to single treatments with either high or low-concentration capsaicin patches for 90, 60 or 30 minutes. Patients were randomized in a 3:3:3:1:1:1 ratio to receive either high-concentration (640 µg/cm² capsaicin) patches for durations of 30, 60 or 90 minutes or low-concentration (3.2 µg/cm² capsaicin) patches for 30, 60 or 90 minutes. The high-concentration and low-concentration patches were of identical appearance. Patients, investigators and sponsor staff were blinded to the treatment received until all data collection activities were completed. Because of prior reports that single applications of low-concentration capsaicin applications do not cause reductions in neuropathic pain, these low-concentration patches were deemed unlikely to exert a significant therapeutic effect. Patches were applied to painful feet areas after a 60-minute topical anesthetic application. Subjects recorded daily pain intensity on an 11-point numeric pain rating scale (0 = no pain, 10 = worst possible pain). The primary efficacy endpoint was the percent change from baseline in mean "average pain for past 24 hours" scores for weeks 2 to 12.

### Assessments

Efficacy in all three studies was assessed using patient-rated pain intensity scores (11-point scale) which were recorded twice daily in take-home diaries during the screening period and the entire 12-week study periods. Each evening, subjects rated their average pain over the preceding 24 hours. The primary efficacy endpoint was the change in the average of morning and evening pain intensity from the baseline period to the follow-up period (average of weeks 2 to 8).

### Patient Demographics

The study population for all three studies represented a wide range of patients with either PHN or HIV-AN. The baseline characteristics of the subjects to which the low-concentration patches were applied in the three studies are shown in Tables 2 - 4.

**Table 2: Baseline Characteristics of Patients in Study 1**

| Number of Subjects with Low-Concentration Patch Applications | 77 |
|---|---|
| Age [years] | |
| Mean ± SD | 71.1 ± 10.4 |
| Range | 27 - 91 |
| Gender | |
| Female | 39 (51%) |
| Male | 38 (49%) |
| Ethnic Origin | |
| Caucasian | 68 (88%) |
| African-American | 1 (1%) |
| Other | 7 (9%) |
| Duration of PHN [years] | |
| Mean ± SD | 3.8 ± 4.5 |
| Range | 0.3 - 21.8 |
| Baseline Pain Level | |
| Mean ± SD | 5.3 ± 1.4 |
| Range | 2.5 - 8.1 |
| Treatment Area Size [cm²] | |
| Mean ± SD | 329 ± 204 |
| Range | 32 - 893 |
| Concomitant Medications | |
| Anticonvulsants | 19 (25%) |
| Antidepressants | 10 (13%) |
| Opioids | 14 (18%) |

| | |
|---|---|
| SD = standard deviation. | |

**Table 3: Baseline Characteristics of Patients in Study 2**

| Number of Subjects with 60-Minute Patch Applications | 53 |
|---|---|
| Age [years] | |
| Mean ± SD | 71.2 ± 11.26 |
| Range | 35,91 |
| Gender | |
| Female | 28 (53%) |
| Male | 25 (47%) |
| Ethnic Origin | |
| Caucasian | 45 (85%) |
| African-American | 3 (6%) |
| Other | 3 (6%) |
| Duration of Pain [years] | |
| Mean ± SD | 3.4 ± 4 |
| Range | 0.3, 19.7 |
| Baseline Pain Level [Average Pain in 24 Hours] | |
| Mean ± SD | 5.3 ± 1.53 |
| Range | 2.5, 8.8 |
| Treatment Area Size [cm²] | |
| Mean ± SD | 348 ± 216 |
| Range | 75,963 |
| Concomitant Medications | |
| Anticonvulsants | 18 (34%) |
| Antidepressants | 6 (11%) |
| Opioids | 13 (25%) |

**Table 4: Baseline Characteristics of Patients in Study 3**

| Number of Patients with Low-Concentration Patch Applications | 82 |
|---|---|
| Age [years] | |
| Mean ± SD | 48.4 ± 7.6 |
| Range | 33 -70 |
| Gender | |
| Female | 3 (4%) |
| Male | 79 (96%) |
| Race | |
| Caucasian | 50 (61%) |
| African-American | 18 (22%) |
| Other | 14 (17%) |
| Duration of HIV-AN [years] | |
| Mean ± SD | 5.1 ± 3.4 |
| Range | 0.1 - 14.2 |
| Baseline Pain Level | |
| Mean ± SD | 5.9 ± 1.6 |
| Range | 2.6 - 9.6 |
| CD4⁺ Count (cells/mm³) | |
| Mean ± SD | 434 ± 280 |
| Median | 406 |
| Range | 12-1373 |
| HIV-1 RNA (log₁₀ copies/mL)^{a} | |
| Mean ± SD | 3.32 ± 0.91 |
| Median | 3.01 |
| Range | 2.60 - 5.82 |
| Neurotoxic Antiretrovirals | |
| Not Taking | 67 (82%) |
| Taking | 15 (18%) |
| Concomitant Pain Medications | |
| Anticonvulsants | 32 (39%) |
| Antidepressants | 31 (38%) |
| Opioids | 15 (18%) |

| | |
|---|---|
| ^{a}For HIV-1 RNA, values less than 400 copies/mL (i.e., below assay limit) are set to 400 (2.60 log₁₀) copies/mL to permit calculation of descriptive statistics. | |

### Results

For Study 1, a total of 299 PHN subjects were enrolled. Of these, 222 were randomly assigned to receive the high-concentration (8% w/w) patch according to the duration of patch application (30, 60, or 90 minutes) and 77 were assigned to receive the low-concentration (0.04% w/w) patch. Overall 24 subjects (8%) terminated prematurely from the study, of which twenty subjects (9%) were in the high-concentration patch group and 4 subjects (5%) in the low-concentration group. Three subjects (1%) terminated due to adverse events in the high-concentration patch group (2 subjects in the 90-minute and 1 subject in the 60-minute group). One subject in the low-concentration group died due to unrelated events of multi-organ failure, 108 days after study drug application. Overall, 275 subjects (91%) completed the double-blind study duration of 12 weeks. All randomized subjects were evaluated for safety and efficacy based on intent-to-treat (ITT) analysis.

For Study 2, a total of 155 PHN subjects were enrolled. Of these, 102 were randomly assigned to receive the high-concentration patch and 53 were assigned to receive the low-concentration patch. Overall 21 subjects (14%) terminated prematurely from the study of which, 11 subjects (11%) were in the active group and 10 subjects (19%) in the low-concentration group. None of the subjects terminated due to adverse events in the study. No deaths were reported in Study 2. Overall, 134 subjects (86%) completed the full study duration of 12 weeks. All 155 randomized subjects were evaluated for safety and efficacy based on intent-to-treat (ITT) analysis.

For Study 3, a total of 307 HIV-AN subjects were enrolled. Patients were randomized in a3:3:3:1:1:1 ratio to receive either high-concentration (640 µg/cm² capsaicin) patches for durations of 30, 60 or 90 minutes or low-concentration (3.2 µg/cm² capsaicin) patches for 30, 60 or 90 minutes. Overall, 274 of 307 subjects (89%) completed the full study duration of 12 weeks. All 307 randomized subjects were evaluated for safety and efficacy based on intent-to-treat (ITT) analysis.

### Efficacy in Study 1

The primary efficacy endpoint was the change from baseline in average pain intensity, as measured on an 11-point scale. The difference between the groups was computed by the difference between the high-concentration patch and the pooled low-concentration patch groups, with baseline pain as covariate. The effect in the low-concentration patch group was much larger than anticipated, particularly for those patients receiving 60-minute patch exposures. In this study, the low-concentration patches produced a significant and sustained decrease in pain.

### Efficacy in Study 2

The mean percent change from baseline in the 'average pain for the past 24 hours" for Weeks 2-8 was -29.9% for subjects treated with the low-concentration patch. The results were consistent across other pain variables. For example, the mean percent change from baseline in the "worst pain for the past 24 hours" for Weeks 2-8 was -27.1 % and the mean percent change from baseline in the "pain now" category for Weeks 2-8 was 31%.

### Efficacy in Study 3

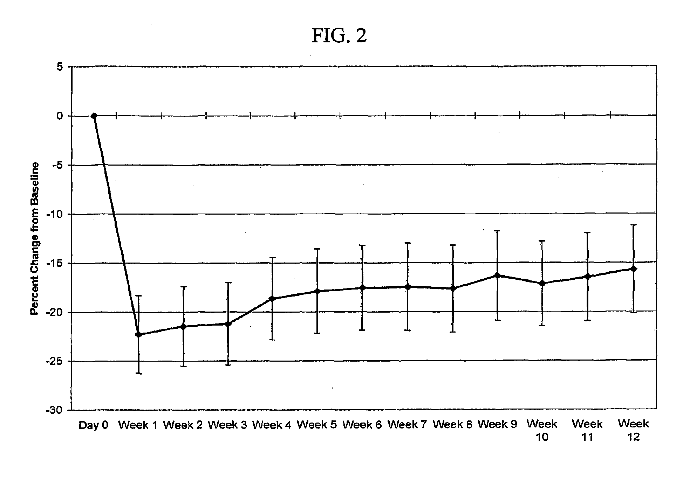

Those patients treated with the low-concentration capsaicin (this includes 30- 60 and 90-minute treatment groups) had an average pain reduction of 11% during weeks 2 to 12 (from baseline of 5.9 to 5.3). See Figure 2.18% of subjects treated with the low-concentration capsaicin patch had ≥ 30% pain decrease from baseline during weeks 2 to 12. Treatment-related adverse events consisted only of mild to moderate local reactions that resolved quickly.

Data from Study 3 were published as an abstract at the 13th Conference on Retroviruses and Opportunistic Infections (2006): "Controlled Study of High-Concentration Capsaicin Patch for Painful HIV-Associated Distal Sensory Polyneuropathy," by D Simpson, S Brown, S Chang, J Jermano and C107 Study Group.

Pooled data from all patients in Studies 1, 2 and 3 receiving low-concentration capsaicin patch treatments is shown in Figure 2. For this graph, a weighted average of pain reduction per week is shown, along with a weighted standard error of the mean. The number of subjects represented by each data point varies between 185 to 208.

From the data generated by Studies 1, 2 and 3 it can be concluded that single treatments of low-concentration capsaicin patches provide long-term pain relief of neuropathic pain in a substantial percentage of patients. Pain relief has been observed in patients with both PHN and HIV-AN; this relief is statistically significant relative to baseline pain values and average pain relief values are only slightly less than induced by high-concentration capsaicin patches. There are no reports in scientific literature of sustained pain relief - lasting for at least 12 weeks - following treatments of neuropathic pain patients with placeboes. Accordingly, it is not plausible that the sustained pain relief observed is due to merely psychological effects. Moreover, low-concentration patches have the added benefits of better tolerability during the treatment procedure and not inducing any systemic capsaicin exposure. Thus overall, the therapeutic index - i.e., the ratio of benefit to adverse events or side effects - of the low-concentration patch is extremely high.

The utility of single topical low-concentration patch treatments is surprising, given current teachings regarding available capsaicin-based products. Although of approximately the same concentration range as over-the-counter products such as Zostrix^{®} cream (0.075%) or Therapatch Warm with Capsaicin^{®} (0.09%), the prescribing information for these and similar products provides no suggestion that single applications of either a cream or patch could provide 12 weeks of analgesic efficacy. In fact, the package insert for Zostix^{®} states that, "For optimum relief apply 3 to 4 times daily. Best results typically occur after 2 to 4 weeks of continuous use." *In vitro* dermal drug delivery studies (conducted by the PRACS Institute, San Diego, CA, USA and Lohmann Therapie-Systeme, Andernach, Germany) have collectively indicated that the amount of capsaicin delivered into deeper skin (dermis) by Zostrix^{®} is substantially lower than that delivered by the low-concentration patch. Accordingly, not all low-concentration products (either patches or creams) behave similarly and some low-concentration patches - particularly those which deliver capsaicin to deeper layers of the skin - may provide sustained pain reductions following single applications. The slow therapeutic response to Zostrix^{®} applications is most likely a manifestation of its poor drug delivery efficiency.

The efficacy of topical applications of capsaicin patches is supported by an emerging understanding of the etiology of peripheral neuropathic pain. Capsaicin-sensitive nerve fibers in the skin are thought to be hyperactive in patients presenting with various peripheral neuropathic pain syndromes (Bley, K.R. Recent developments in transient receptor potential vanilloid receptor 1 agonist-based therapies. Expert Opin Investig Drugs. 2004. 13:1445-56.). Consequently, topical applications of capsaicin have long been recognized as a treatment option, due to the ability of capsaicin to inhibit nociceptor hyperactivity (Bley, K.R. Recent developments in transient receptor potential vanilloid receptor 1 agonist-based therapies. 2004. Expert Opin Investig Drugs*.).* This process of long-term nociceptor inhibition is known as "desensitization", and is the goal of topical capsaicin therapy (Szallasi A, Blumberg PM. Vanilloid (capsaicin) receptors and mechanisms. Pharmacol. Rev. 1999. 51:159-212).

Due to the large number of patients treated, the data from Studies 1, 2 and 3 provide strong evidence for the efficacy of the low-concentration patch. Moreover, subject with two etiologically distinct neuropathic pain syndromes were enrolled in these trials; this is suggestive of a broad-based efficacy against neuropathic pain.

The amount or dose of capsaicin which needs to be delivered into the skin for desensitization to occur is likely to vary between patients. The nervous system is very plastic, so following the multitude of injuries or lesions which can produce neuropathic pain, it is expected that the peripheral nervous system will respond in a variety of ways. One observed consequence is that nerve fibers which remain in the skin following neuropathic injury become hyperactive due to overexposure to neurotrophic factors and the subsequent expression of pro-excitatory proteins. TRPV1, the capsaicin receptor, is one of these pro-excitatory proteins. Consequently, in the cutaneous nerve fibers of some patients with peripheral neuropathic pain syndromes, TRPV1 over-expression may lead to dramatically increased sensitivity to capsaicin. Therefore capsaicin-induced desensitization could be initiated by much lower concentrations or doses of capsaicin than previously expected.

## Claims

1. A composition comprising capsaicin or a capsaicin analog for use in the treatment of neuropathic pain, wherein the composition is provided as a patch comprising:
capsaicin or a capsaicin analog, wherein the capsaicin analog is selected from dihydrocapsaicin, nordihydrocapsaicin, homocapsaicin, homodihydrocapsaicin, nonivamide, cis-capsaicin, olvanil, arvanil and capsaicin esters, wherein the concentration of the capsaicin or capsaicin analog is less than 1% by weight;
a penetration enhancer, wherein the penetration enhancer is diethylene glycol monoethyl ether; and
a backing layer;
and wherein the patch is administered in a single application of not longer than a period of 120 minutes to provide relief of neuropathic pain for at least one month.

2. The composition of claim 1, wherein the patch provides relief of neuropathic pain for at least two months.

3. The composition of claim 2, wherein the patch provides relief of neuropathic pain for at least three months.

4. The composition of any one of the preceding claims wherein the patch further comprises a self-adhesive matrix.

5. The composition of claim 4, wherein the self-adhesive matrix comprises an amine-resistant polysiloxane.

6. The composition of claim 5, wherein the amine resistant polysiloxane comprises a mixture of medium and high tack polysiloxane.

7. The composition of any one of the preceding claims, wherein the patch further comprises about 0.5% to about 5% by weight of a silicone oil.

8. The composition of any one of the preceding claims, wherein the patch further comprises a viscosity increasing agent.

9. The composition of claim 8, wherein the viscosity increasing agent is ethylcellulose, hydropropylcellulose, or mixtures thereof

10. A composition according to claim 1, wherein the patch comprises:
0.04% by weight or less of capsaicin or a capsaicin analog, wherein the capsaicin analog is selected from dihydrocapsaicin, nordihydrocapsaicin, homocapsaicin, homodihydrocapsaicin, nonivamide, cis-capsaicin, olvanil, arvanil, capsaicin esters, and derivatives of the amide side chain of capsaicin;
10-20% by weight of diethylene glycol monoethyl ether;
0-2% by weight of ethylcellulose;
0-5% by weight of silicone oil; and
58-85% by weight of a self adhesive polysiloxane.

11. The composition of any one of the preceding claims, wherein the backing layer comprises a polyester film.

12. The composition of any one of the preceding claims, wherein the backing layer is about 10 to about 20 µm thick.

13. The composition according to any one of claims 1 to 12 for use in treating neuropathic pain by applying the patch to an area of skin.

## Patentansprüche

1. Zusammensetzung, die Capsaicin oder ein Capsaicin-Analogon umfasst, zur Verwendung in der Behandlung von neuropathischem Schmerz, wobei die Zusammensetzung als ein Pflaster bereitgestellt wird, das umfasst:
Capsaicin oder ein Capsaicin-Analogon, wobei das Capsaicin-Analogon aus Dihydrocapsaicin, Nordihydrocapsaicin, Homocapsaicin, Homodihydrocapsaicin, Nonivamid, Cis-Capsaicin, Olvanil, Arvanil und Capsaicin-Estern ausgewählt ist, wobei die Konzentration des Capsaicins oder Capsaicin-Analogons weniger als 1 Gew.-% ist;
einen Penetrationsverstärker, wobei der Penetrationsverstärker Diethylenglycolmonoethylether ist, und
eine Trägerschicht;
und wobei das Pflaster in einer einzelnen Anwendung nicht länger als einen Zeitraum von 120 Minuten verabreicht wird, um eine Linderung von neuropathischem Schmerz für wenigstens einen Monat bereitzustellen.

2. Zusammensetzung gemäß Anspruch 1, wobei das Pflaster eine Linderung von neuropathischem Schmerz für wenigstens zwei Monate bereitstellt.

3. Zusammensetzung gemäß Anspruch 2, wobei das Pflaster eine Linderung von neuropathischem Schmerz für wenigstens drei Monate bereitstellt.

4. Zusammensetzung gemäß einem der vorangehenden Ansprüche, wobei das Pflaster außerdem eine selbstklebende Matrix umfasst.

5. Zusammensetzung gemäß Anspruch 4, wobei die selbstklebende Matrix ein Amin-resistentes Polysiloxan umfasst.

6. Zusammensetzung gemäß Anspruch 5, wobei das Aminresistente Polysiloxan ein Gemisch aus Polysiloxan mit mittlerer und hoher Klebrigkeit umfasst.

7. Zusammensetzung gemäß einem der vorangehenden Ansprüche, wobei das Pflaster außerdem etwa 0,5 bis etwa 5 Gew.-% eines Silikonöls umfasst.

8. Zusammensetzung gemäß einem der vorangehenden Ansprüche, wobei das Pflaster außerdem ein die Viskosität erhöhendes Mittel umfasst.

9. Zusammensetzung gemäß Anspruch 8, wobei das die Viskosität erhöhende Mittel Ethylcellulose, Hydropropylcellulose oder Gemische davon ist.

10. Zusammensetzung gemäß Anspruch 1, wobei das Pflaster umfasst:
0,04 Gew.-% oder weniger an Capsaicin oder einem Capsaicin-Analogon, wobei das Capsaicin-Analogon aus Dihydrocapsaicin, Nordihydrocapsaicin, Homocapsaicin, Homodihydrocapsaicin, Nonivamid, Cis-Capsaicin, Olvanil, Arvanil, Capsaicin-Estern und Derivaten der Amidseitenkette von Capsaicin ausgewählt ist;
10-20 Gew.-% Diethylenglycolmonoethylether;
0-2 Gew.-% Ethylcellulose;
0-5 Gew.-% Silikonöl und
58-85 Gew.-% eines selbstklebenden Polysiloxans.

11. Zusammensetzung gemäß einem der vorangehenden Ansprüche, wobei die Trägerschicht einen Polyesterfilm umfasst.

12. Zusammensetzung gemäß einem der vorangehenden Ansprüche, wobei die Trägerschicht etwa 10 bis etwa 20 µm dick ist.

13. Zusammensetzung gemäß einem der Ansprüche 1 bis 12 zur Verwendung bei der Behandlung von neuropathischem Schmerz durch Auftragen des Pflasters auf einen Hautbereich.

## Revendications

1. Composition comprenant de la capsaïcine, ou un analogue de capsaïcine, pour utilisation dans le traitement d'une douleur neuropathique, laquelle composition est fournie sous la forme d'un timbre comprenant :
- de la capsaïcine ou un analogue de capsaïcine, étant entendu que cet analogue de capsaïcine est choisi parmi la dihydrocapsaïcine, la nordihydrocapsaïcine, l'homocapsaïcine, l'homodihydrocapsaïcine, le nonivamide, la cis-capsaïcine, l'olvanil, l'arvanil et les esters de capsaïcine, et que la concentration de capsaïcine ou d'analogue de capsaïcine est inférieure à 1 % en poids,
- un agent favorisant la pénétration, lequel agent favorisant la pénétration est de l'éther monoéthylique de diéthylèneglycol,
- et une couche de dos,
et est administrée en une application unique du timbre ne durant pas plus de 120 minutes, afin de procurer un soulagement de la douleur neuropathique pour au moins un mois.

2. Composition conforme à la revendication 1, avec laquelle le timbre procure un soulagement de la douleur neuropathique pour au moins deux mois.

3. Composition conforme à la revendication 2, avec laquelle le timbre procure un soulagement de la douleur neuropathique pour au moins trois mois.

4. Composition conforme à l'une des revendications précédentes, dans laquelle le timbre comporte en outre une matrice auto-adhésive.

5. Composition conforme à la revendication 4, dans laquelle la matrice auto-adhésive comprend un polysiloxane résistant aux amines.

6. Composition conforme à la revendication 5, dans laquelle le polysiloxane résistant aux amines comprend un mélange de polysiloxanes à forte adhésivité et à moyenne adhésivité.

7. Composition conforme à l'une des revendications précédentes, dans laquelle le timbre comporte en outre une huile de silicone, en une proportion d'environ 0,5 % à environ 5 % en poids.

8. Composition conforme à l'une des revendications précédentes, dans laquelle le timbre comporte en outre un agent augmentant la viscosité.

9. Composition conforme à la revendication 8, dans laquelle l'agent augmentant la viscosité est de l'éthyl-cellulose, de l'hydroxy-propyl-cellulose, ou un mélange de ces corps.

10. Composition conforme à la revendication 1, dans laquelle le timbre comprend :
- 0,04 % en poids ou moins de capsaïcine ou d'un analogue de capsaïcine, étant entendu que l'analogue de capsaïcine est choisi parmi la dihydrocapsaïcine, la nordihydrocapsaïcine, l'homocapsaïcine, l'homodihydrocapsaïcine, le nonivamide, la cis-capsaïcine, l'olvanil, l'arvanil, les esters de capsaïcine, et les dérivés de capsaïcine formés au niveau de la chaîne latérale à fonction amide,
- de 10 à 20 % en poids d'éther monoéthylique de diéthylèneglycol,
- de 0 à 2 % en poids d'éthyl-cellulose,
- de 0 à 5 % en poids d'une huile de silicone,
- et de 58 à 85 % en poids d'un polysiloxane auto-adhésif.

11. Composition conforme à l'une des revendications précédentes, dans laquelle la couche de dos comprend un film en polyester.

12. Composition conforme à l'une des revendications précédentes, dans laquelle la couche de dos est épaisse d'environ 10 µm à environ 20 µm.

13. Composition conforme à l'une des revendications 1 à 12, pour utilisation dans le traitement d'une douleur neuropathique par application du timbre sur une région de la peau.
